# FASCICULE DE BREVET EUROPEEN

(11) **EP 1 545 354 B1**
(45) Date de publication et mention de la délivrance du brevet: **26.04.2006**
(21) Numéro de dépôt: 03769561.6
(22) Date de dépôt: 03.09.2003
(51) Int. Cl.: A61B 17/70, A61F 2/44

(54) **ENSEMBLE DE SOUTIEN VERTEBRAL POSTERIEUR**
POSTERIORE WIRBELSTÜTZANORDNUNG
POSTERIOR VERTEBRAL SUPPORT ASSEMBLY

(30) Priorité: 10.09.2002 FR 0211189
(43) Date de publication de la demande: 29.06.2005
(73) Titulaire: Taylor, Jean, 06400 Cannes (FR)
(72) Inventeur: Taylor, Jean, 06400 Cannes (FR)
(74) Mandataire: Neyret, Daniel Jean Marie
(86) Numéro de dépôt international: PCT/FR2003/002635
(87) Numéro de publication internationale: WO 2004/024010

(56) Documents cités:
- FR-A- 2 722 088
- FR-A- 2 799 640
- FR-A- 2 811 540
- FR-A- 2 818 530
- US-B1- 6 402 750

## Description

La présente invention concerne un ensemble de soutien vertébral postérieur.

En cas de dégénérescence du disque intervertébral de deux vertèbres et/ou de distension ligamentaire, il est connu de placer une cale entre les apophyses épineuses des deux vertèbres concernées, permettant de soutenir les vertèbres. On pourra à ce propos se reporter aux demandes de brevet français FR-A-2.717.675 et FR-A-2.775.183 (Le préambule de la revendication 1 est basé sur ce document.) déposées au nom du titulaire de la présente demande.

Les cales selon ces demandes de brevet antérieures sont reliées aux apophyses épineuses des deux vertèbres traitées par deux ligaments indépendants, chacun de ces ligaments traversant la cale et entourant l'apophyse épineuse correspondante de manière serrée. En cas de flexion du rachis vers l'avant, les apophyses épineuses s'écartent l'une de l'autre, ce qui provoque un étirement longitudinal de la cale.

Cet étirement longitudinal a pour inconvénient de solliciter la cale dans le sens d'une traction longitudinale. Les conditions d'exercice de cette traction sont toutefois perfectibles, en particulier afin d'obtenir un contrôle assisté et encadré du mouvement des vertèbres, et ce d'autant que la répétition de cette traction menace d'affecter la pérennité de la cale.

La présente invention vise à remédier à cet inconvénient.

L'ensemble qu'elle concerne comprend, de manière connue en soi, une cale interépineuse conformée pour pouvoir être insérée entre les apophyses épineuses de deux vertèbres à traiter, dont au moins la zone destinée à être placée entre les apophyses épineuses des vertèbres est en un matériau élastiquement déformable.

Selon l'invention, l'ensemble comprend en outre :
- deux éléments latéraux de compression, destinés à être placés de part et d'autre de la cale dans le sens longitudinal, ces éléments latéraux de compression étant déformables entre des positions de relâchement, qu'ils occupent lorsque les vertèbres sont en lordose ou lorsque le rachis est en extension, dans lesquelles ils sont comparativement éloignés de la cale dans le sens transversal, et des positions de compression, qu'ils occupent lorsque le rachis est en flexion, dans lesquelles ils sont comparativement rapprochés de la cale dans le sens transversal ; et
- deux éléments latéraux de transmission, placés entre les éléments latéraux de compression et la cale, conformés pour, lorsque les éléments latéraux de compression sont déplacés dans ladite position de compression, appuyer contre la cale dans le sens transversal de celle-ci, à hauteur de la zone de la cale destinée à être placée entre les apophyses épineuses des vertèbres.

L'ensemble selon l'invention permet ainsi d'exercer une compression transversale progressive sur la cale au cours de mouvement de flexion du rachis. Cette compression vient réduire les contraintes en cisaillement qui s'exercent sur la cale lors d'un écartement intervertébral combinant des mouvements de bascule et glissement antérieur, grâce à l'encadrement des déplacements qu'opère la cale.

La compression transversale de la cale est préférable à une pure traction longitudinale du point de vue de la pérennité de la cale, étant donné qu'elle compense la sollicitation de la cale dans le sens longitudinal.

La zone de la cale destinée à être placée entre les apophyses épineuses peut être en un matériau ayant une limite de compressibilité dans le sens transversal de la cale, et l'ensemble peut alors être conformé de telle sorte que cette limite soit atteinte lorsque les vertèbres traitées atteignent une position prédéterminée de bascule.

Il est également possible de conformer les éléments latéraux de compression de manière à ce que ces éléments aient une limite de déformation dans le sens transversal, cette limite de déformation étant atteinte lorsque les vertèbres traitées atteignent une position prédéterminée de bascule.

Les éléments latéraux de compression peuvent être déformés de manière non élastique ou de manière élastique entre lesdites positions de relâchement et de compression. Dans ce deuxième cas, la force de rappel de ces éléments vers leur forme neutre contribue à l'amortissement du mouvement de bascule des vertèbres traitées.

Ces éléments latéraux de compression peuvent également être déformables de manière élastique dans le sens longitudinal de ces éléments latéraux de compression, dans le même but.

Selon une forme de réalisation de l'invention, les éléments latéraux de compression sont indépendants l'un de l'autre, et chacun d'eux est relié à l'une des vertèbres traitées par une extrémité et à l'autre vertèbre traitée par son autre extrémité. La liaison des extrémités des éléments latéraux de compression aux vertèbres peut notamment se faire au niveau des pédicules des vertèbres, au moyen des vis pédiculaires engagées dans des oeillets ou des pièces d'ancrage que comprennent les éléments latéraux de compression.

La liaison des éléments latéraux de compression aux vertèbres peut également se faire, s'agissant de la vertèbre sus-jacente, par le biais d'un passage des éléments sous les lames de cette vertèbre. Ce passage sous lamaire permet de préserver les pédicules et d'être le plus médian possible pour optimiser, lors de l'élongation longitudinale, la poussée transversale exercée sur la cale. S'agissant de la vertèbre sous-jacente, ces mêmes éléments latéraux de compression peuvent également être connectés à une barre de liaison transversale interpédiculaire, mise en place notamment en cas de laminectomie sur cette vertèbre, permettant ainsi de réduire la concentration de contraintes. De même, les éléments latéraux de compression peuvent être connectés à une barre de liaison reliée à un système d'arthrodèse des deux vertèbres sous-jacentes.

Selon une autre forme de réalisation de l'invention, les éléments latéraux de compression sont formés par les deux parties latérales d'un lien circulaire engagé autour des apophyses épineuses des deux vertèbres traitées.

Les éléments latéraux de transmission peuvent quant à eux être constitués par des barrettes prenant appui contre les éléments latéraux de compression d'une part et contre la cale, d'autre part, notamment par l'intermédiaire de plaquettes d'appui.

Ces mêmes éléments latéraux de transmission peuvent également être constitués de bossages reliés soit aux éléments latéraux de compression soit à la cale. Dans ce deuxième cas, ces bossages peuvent former corps avec la cale.

La cale peut être réalisée entièrement en un matériau élastiquement déformable tel qu'un silicone ; elle peut notamment comprendre un noyau en un tel matériau et une enveloppe textile contenant ce noyau.

La cale peut également être formée par une bande de matériau élastique, convenablement recourbé.

La cale peut comprendre un ressort placé transversalement au niveau de sa zone destinée à être placée entre les apophyses épineuses.

Pour sa bonne compréhension, l'invention est à nouveau décrite ci-dessous en référence au dessin schématique annexé représentant, à titre d'exemple non limitatif, plusieurs formes de réalisation possibles de l'ensemble qu'elle concerne.
La figure 1 en est une vue postérieure, très simplifiée, après mise en place sur deux vertèbres ;
la figure 2 en est une vue similaire à la figure 1, selon la deuxième forme de réalisation ;
la figure 3 en est une vue similaire à la figure 1 selon la troisième forme de réalisation, seules les apophyses épineuses des vertèbres étant représentées, et
la figure 4 en est une vue similaire à la figure 3, selon une variante.

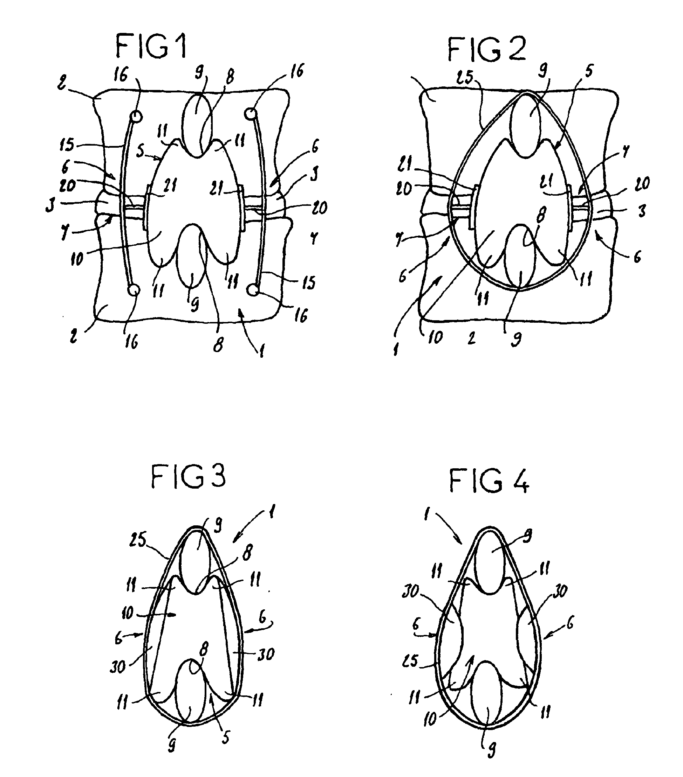

La figure 1 représente un ensemble 1 de soutien vertébral postérieur mis en place sur deux vertèbres 2 subissant une dégénérescence du disque 3 et/ou une distension ligamentaire.

L'ensemble 1 comprend une cale interépineuse 5, deux éléments latéraux de compression 6 et deux éléments latéraux de transmission 7.

La cale 5 est en un matériau souple élastiquement et comprend deux échancrures 8 lui permettant d'être insérée entre les apophyses épineuses 9 des deux vertèbres 2. Elle peut notamment être conforme à la cale selon la demande de brevet FR-A-2.775.183, c'est-à-dire :
- comprenant un noyau en silicone et une enveloppe en tissu de polyester,
- présentant une partie interépineuse 10 ayant une hauteur supérieure à la distance séparant les apophyses épineuses 9 lorsque les vertèbres 2 sont en lordose afin d'être comprimée lorsque la cale 5 est insérée entre ces apophyses 9 et de permettre un soulagement du disque 3 ainsi qu'une remise en tension fonctionnelle des ligaments, et
- présentant des oreilles 11 qui délimitent les échancrures 8, ces oreilles 11 enveloppant largement les apophyses 9 et étant auto-serrées autour de ces apophyses 9 lorsque ladite partie interépineuse 10 est comprimée.

La cale 5 montrée sur la figure 1 diffère toutefois de la cale selon la demande de brevet FR-A-2.775.183 précitée par le fait qu'elle présente des parois latérales bombées, lui donnant une largeur relativement importante au niveau de ladite partie interépineuse 10.

Chaque élément 6 est formé par une lame 15 en matière synthétique élastiquement déformable et par deux oeillets 16 reliés aux extrémités de la lame 15. Cette dernière est courbe et comprend l'élément 7 au niveau de la partie médiane de sa face concave, cette face concave étant destinée à être tournée vers la cale 5.

Les oeillets 16 sont destinés à recevoir des vis pédiculaires permettant l'ancrage des éléments 6 aux vertèbres 2.

Chaque élément 7 est formé par une barrette rigide 20 fixée à un élément 6, et est orientée sensiblement perpendiculairement à la zone de l'élément 6 à laquelle elle est reliée. Cette barrette 20 est placée, après implantation, à la hauteur de la partie inter-épineuses 10 de la cale 5.

Chaque barrette 20 est solidaire d'une plaquette 21 d'appui contre cette partie 10.

L'ensemble 1 selon l'invention se trouve dans la position montrée sur la figure 1 lorsque les vertèbres 2 sont en lordose.

En cas d'extension du rachis, les vertèbres 2 pivotent dans le sens du rapprochement mutuel des apophyses 9, ce qui amène à une compression de la cale 5 jusqu'à la limite élastique du matériau constituant le noyau de cette cale. Un amortissement du mouvement de pivotement des vertèbres 2 est ainsi obtenu tant que ladite limite élastique n'est pas atteinte, puis un arrêt de ce même mouvement est obtenu lorsque cette limite élastique est atteinte. Lors de cette compression de la cale 5, les éléments 6 se déforment dans le sens de l'augmentation de leur courbure contribuent à l'amortissement du mouvement des vertèbres 2. Ces éléments 6 assurent également une parfaite garantie du maintien en position de la cale 5 entre les apophyses 9.

En cas de flexion du rachis, les vertèbres 2 pivotent dans le sens de l'éloignement mutuel des apophyses 9, ce qui amène à une réduction dé la courbure des éléments 6. Les éléments 7 appuient alors contre la cale 5 transversalement, ce qui permet également d'amortir le mouvement des vertèbres 2 puis d'arrêter ce mouvement lorsque la limite élastique du matériau formant le noyau de la cale 5 est atteint et/ou lorsque les éléments 6 s'approchent d'une forme sensiblement rectiligne.

La figure 2 montre un ensemble 1 similaire à celui qui vient d'être décrit sinon que les éléments 6 sont formées par les deux parties latérales d'un lien circulaire 25 engagé autour des apophyses 9 des vertèbres 2. Les autres parties ou éléments déjà décrits, qui se retrouvent dans cette deuxième forme de réalisation, ne sont pas décrits à nouveau et sont désignés par les mêmes références numériques que précédemment.

Le lien 25 peut être en un matériau légèrement étirable élastiquement, et contribue ainsi également à l'amortissement puis au blocage du mouvement de pivotement des vertèbres 2 en cas de flexion du rachis.

Les figures 3 et 4 montrent une troisième forme de réalisation de l'ensemble 1, dans laquelle ce dernier est similaire à celui montré par la figure 2 sinon que les barrettes 20 et plaquettes 21 sont remplacées par des bossages 30 le long desquels passent les parties latérales du lien 25. Ces bossages 30 peuvent être fixés à la cale 5 ou peuvent former corps avec elle, comme montré sur la figure 3, ou peuvent être solidaires du lien 25 et prendre appui contre la cale 5, éventuellement au niveau d'évidements latéraux que celle-ci comprend pour les recevoir, comme montré sur la figure 4.

Ainsi qu'il apparaît de ce qui précède, l'invention apporte une amélioration déterminante à la technique antérieure, en fournissant un ensemble de soutien vertébral postérieur qui ne sollicite pas la cale dans le sens longitudinal lors du mouvement de flexion du rachis. Un parfait contrôle du mouvement des vertèbres est obtenu au moyen de cet ensemble, et la pérennité de la cale n'est pas affectée par les mouvements répétés des vertèbres.

Il va de soi que l'invention n'est pas limitée à la forme de réalisation décrite ci-dessus à titre d'exemple mais qu'elle en embrasse au contraire toutes les variantes de réalisation entrant dans le champ de protection défini par les revendications ci-annexées.

## Revendications

1. Ensemble (1) de soutien vertébral postérieur, comprenant une cale interépineuse (5) conformée pour pouvoir être insérée entre les apophyses épineuses (9) de deux vertèbres (2) à traiter, dont au moins la zone destinée à être placée entre les apophyses épineuses des vertèbres est en un matériau élastiquement déformable ;
ensemble (1) **caractérisé en ce qu'**il comprend :
- deux éléments latéraux de compression (6), destinés à être placés de part et d'autre de la cale (5) dans le sens longitudinal, ces éléments latéraux de compression (6) étant déformables entre des positions de relâchement, qu'ils occupent lorsque les vertèbres (2) sont en lordose ou lorsque le rachis est en extension, dans lesquelles ils sont comparativement éloignés de la cale (5) dans le sens transversal, et des positions de compression, qu'ils occupent lorsque le rachis est en flexion, dans lesquelles ils sont comparativement rapprochés de la cale (5) dans le sens transversal ; et
- deux éléments latéraux de transmission (7), placés entre les éléments latéraux de compression (6) et la cale (5), conformés pour, lorsque les éléments latéraux de compression (6) sont déplacés dans ladite position de compression, appuyer contre la cale (5) dans le sens transversal de celle-ci, à hauteur de la zone (10) de la cale (5) destinée à être placée entre les apophyses épineuses (9) des vertèbres (2).

2. Ensemble (1) de soutien selon la revendication 1, **caractérisé en ce que** le matériau constitutif de la zone de la cale (5) destinée à être placée entre les apophyses épineuses a une limite de compressibilité dans le sens transversal de la cale (5), et **en ce que** cet ensemble (1) est conformé de telle sorte que cette limite soit atteinte lorsque les vertèbres (2) traitées atteignent une position prédéterminée de bascule.

3. Ensemble (1) de soutien selon la revendication 1 ou la revendication 2, **caractérisé en ce que** les éléments latéraux de compression (6) sont conformés de manière à avoir une limite de déformation dans le sens transversal, cette limite de déformation étant atteinte lorsque les vertèbres (2) traitées atteignent une position prédéterminée de bascule.

4. Ensemble (1) de soutien selon l'une des revendications 1 à 3, **caractérisé en ce que** les éléments latéraux de compression (6) sont conformés de manière à être déformables élastiquement entre lesdites positions de relâchement et de compression.

5. Ensemble (1) de soutien selon l'une des revendications 1 à 4, **caractérisé en ce que** les éléments latéraux de compression (6) sont conformés de manière à être déformables élastiquement dans le sens longitudinal de ces éléments latéraux de compression (6).

6. Ensemble (1) de soutien selon l'une des revendications 1 à 5, **caractérisé en ce que** les éléments latéraux de compression (6) sont indépendants l'un de l'autre, et **en ce que** chacun d'eux est relié à l'une des vertèbres (2) traitées par une extrémité et à l'autre vertèbre (2) traitée par son autre extrémité.

7. Ensemble (1) de soutien selon la revendication 6, **caractérisé en ce que** les éléments latéraux de compression (6) comprennent des oeillets (16) ou des pièces d'ancrage destinés à recevoir des vis pédiculaires d'ancrage de ces éléments latéraux de compression (6) aux vertèbres (2).

8. Ensemble (1) de soutien selon l'une des revendications 1 à 5, **caractérisé en ce que** les éléments latéraux de compression sont conformés de manière à pouvoir passer sous les lames de la vertèbre sus-jacente.

9. Ensemble (1) de soutien selon l'une des revendications 1 à 5, **caractérisé en ce que** les éléments latéraux de compression sont conformés de manière à pouvoir être connectés à une barre de liaison transversale interpédiculaire, mise en place sur la vertèbre sous-jacente notamment en cas de laminectomie.

10. Ensemble (1) de soutien selon l'une des revendications 1 à 5, **caractérisé en ce que** les éléments latéraux de compression sont conformés de manière à pouvoir être connectés à une barre de liaison reliée à un système d'arthrodèse des deux vertèbres sous-jacentes.

11. Ensemble (1) de soutien selon l'une des revendications 1 à 5, **caractérisé en ce que** les éléments latéraux de compression (6) sont formés par les deux parties latérales d'un lien circulaire (25) engagé autour des apophyses épineuses (9) des deux vertèbres (2) traitées.

12. Ensemble (1) de soutien selon l'une des revendications 1 à 11, **caractérisé en ce que** les éléments latéraux de transmission (7) sont constitués par des barrettes (20) prenant appui contre les éléments latéraux de compression (6) d'une part et contre la cale (5), d'autre part.

13. Ensemble (1) de soutien selon l'une des revendications 1 à 11, **caractérisé en ce que** les éléments latéraux de transmission (7) sont constitués de bossages (30) reliés soit aux éléments latéraux de compression (6) soit à la cale (5).

## Claims

1. Rear vertebral support assembly (1), comprising an interspinous wedge (5) which is shaped such that it can be inserted between the spiny apophyses (9) of two vertebrae (2) to be treated, of which at least the area which is destined to be placed between the spiny apophyses of the vertebrae is made of a resiliently deformable material,
which assembly (1) is **characterised in that** it comprises:
- two lateral compression elements (6) which are designed to be placed on both sides of the wedge (5) in the longitudinal direction, these lateral compression elements (6) being deformable between positions of release, which they occupy when the vertebrae (2) are in lordosis, or when the rachis is extended, in which they are comparatively distant from the wedge (6) in the transverse direction, and positions of compression, which they occupy when the rachis is undergoing flexion, in which they are comparatively close to the wedge (5) in the transverse direction; and
- two lateral transmission elements (7), which are placed between the lateral compression elements (6) and the wedge (5), and are shaped such that, when the lateral compression elements (6) are displaced into the said compression position, they are supported against the wedge (5) in the transverse direction of the latter, at the level of the area (10) of the wedge (5) which is designed to be placed between the spiny apophyses (9) of the vertebrae (2).

2. Support assembly (1) according to claim 1, **characterised in that** the material which constitutes the area of the wedge (5) which is designed to be placed between the spiny apophyses has a limit of compressibility in the transverse direction of the wedge (5), and **in that** this assembly (1) is shaped such that this limit is reached when the vertebrae (2) treated reach a predetermined tilting position.

3. Support assembly (1) according to claim 1 or claim 2, **characterised in that** the lateral compression elements (6) are shaped such as to have a limit of deformation in the transverse direction, this limit of deformation being reached when the vertebrae treated (2) reach a predetermined tilting position.

4. Support assembly (1) according to any one of claims 1 to 3, **characterised in that** the lateral compression elements (6) are shaped such as to be deformable resiliently between the said positions of release and compression.

5. Support assembly (1) according to any one of claims 1 to 4, **characterised in that** the lateral compression elements (6) are shaped such as to be deformable resiliently in the longitudinal direction of these lateral compression elements (6).

6. Support assembly (1) according to any one of claims 1 to 5, **characterised in that** the lateral compression elements (6) are independent from one another, and **in that** each of them is connected to one of the vertebrae treated (2) by one end, and to the other vertebra treated (2) by its other end.

7. Support assembly (1) according to claim 6, **characterised in that** the lateral compression elements (6) comprise eyelets (16) or anchorage parts which are designed to receive pedicular screws for anchorage of these lateral compression elements (6) to the vertebrae (2).

8. Support assembly (1) according to any one of claims 1 to 5, **characterised in that** the lateral compression elements are shaped such as to be able to pass beneath the laminae of the underlying vertebra.

9. Support assembly (1) according to any one of claims 1 to 5, **characterised in that** the lateral compression elements are shaped such as to be able to be connected to an inter-pedicular transverse connection bar which is put into place on the underlying vertebra, in particular in the case of a laminectomy.

10. Support assembly (1) according to any one of claims 1 to 5, **characterised in that** the lateral compression elements are shaped such as to be able to be attached to a connection bar which is connected to a system for arthrodesis of the two underlying vertebrae.

11. Support assembly (1) according to any one of claims 1 to 5, **characterised in that** the lateral compression elements (6) are formed by the two lateral parts of a circular link (25) which is engaged around the spiny apophyses (9) of the two vertebrae treated (2).

12. Support assembly (1) according to any one of claims 1 to 11, **characterised in that** the lateral transmission elements (7) consist of strips (20) which are supported on the one hand against the lateral compression elements (6) and on the other hand against the wedge (5).

13. Support assembly (1) according to any one of claims 1 to 11, **characterised in that** the lateral transmission elements (7) consist of bosses (30) which are connected either to the lateral compression elements (6) or to the wedge (5).

## Patentansprüche

1. Anordnung (1) zur posterioren Wirbelstützung, bestehend aus einem, zum Einfügen zwischen die Dornfortsätze (9) zweier zu behandelnder Wirbel (2) geformten, interspinalen Block (5), bei dem mindestens der Bereich, der dafür bestimmt ist, zwischen den Dornfortsätzen der Wirbel angeordnet zu werden, aus einem elastisch verformbaren Material besteht,
Anordnung (1), **dadurch gekennzeichnet, dass** sie umfasst
- zwei seitliche Kompressionselemente (6), die dazu bestimmt sind, in Längsrichtung beiderseits des Blockes (5) angeordnet zu werden, wobei diese seitlichen Kompressionselemente (6) zwischen Entspannungsstellungen, die sie einnehmen, wenn sich die Wirbel (2) in Lordose befinden oder die Wirbelsäule gestreckt ist, in denen sie vom Block (5) in Querrichtung relativ weit entfernt sind, und Kompressionsstellungen, die sie einnehmen, wenn die Wirbelsäule gebeugt ist, in denen sie dem Block (5) in Querrichtung relativ nahe sind, verformbar sind, und
- zwei seitliche Übertragungsteile (7), die zwischen den seitlichen Kompressionselementen (6) und dem Block (5) angeordnet sind, derart ausgebildet, dass sie, wenn die seitlichen Kompressionselemente (6) in die genannte Kompressionsstellung versetzt sind, in Höhe des Abschnittes (10) des Blockes (5) in dessen Querrichtung auf den genannten Block (5) drücken, der dafür bestimmt ist, zwischen die Dornfortsätze (9) der Wirbel (2) eingefügt zu werden.

2. Stützanordnung (1) nach Patentanspruch 1, **dadurch gekennzeichnet, dass** das Material, aus dem der Abschnitt des Blockes (5) besteht, der dafür bestimmt ist, zwischen die Dornfortsätze eingefügt zu werden, eine Komprimierbarkeitsgrenze in der Querrichtung des Blockes (5) aufweist, und **dadurch**, dass diese Anordnung (1) derart ausgestaltet ist, dass diese Grenze erreicht wird, wenn die behandelten Wirbel (2) eine festgelegte Kippstellung erreichen.

3. Stützanordnung (1) nach Patentanspruch 1 oder Patentanspruch 2, **dadurch gekennzeichnet, dass** die seitlichen Kompressionselemente (6) derart ausgestaltet sind, dass sie eine Verformungsgrenze in Querrichtung aufweisen, wobei diese Verformungsgrenze erreicht wird, wenn die behandelten Wirbel (2) eine festgelegte Kippstellung erreichen.

4. Stützanordnung (1) nach einem der Patentansprüche 1 bis 3, **dadurch gekennzeichnet, dass** die seitlichen Kompressionselemente (6) derart ausgestaltet sind, dass sie zwischen den genannten Entspannungs- und Kompressionsstellungen elastisch verformbar sind.

5. Stützanordnung (1) nach einem der Patentansprüche 1 bis 4, **dadurch gekennzeichnet, dass** die seitlichen Kompressionselemente (6) derart ausgestaltet sind, dass sie in der Längsrichtung dieser seitlichen Kompressionselemente (6) elastisch verformbar sind.

6. Stützanordnung (1) nach einem der Patentansprüche 1 bis 5, **dadurch gekennzeichnet, dass** die seitlichen Kompressionselemente (6) voneinander unabhängig sind und **dadurch**, dass jedes von ihnen an einem Ende mit einem der behandelten Wirbel (2) verbunden ist und an seinem anderen Ende mit dem anderen Wirbel (2).

7. Stützanordnung (1) nach Patentanspruch 6, **dadurch gekennzeichnet, dass** die seitlichen Kompressionselemente (6) Ösen (16) oder Verankerungsteile aufweisen, die dazu bestimmt sind, Pedikelschrauben zur Verankerung dieser seitlichen Kompressionselemente (6) an den Wirbeln (2) aufzunehmen.

8. Stützanordnung (1) nach einem der Patentansprüche 1 bis 5, **dadurch gekennzeichnet, dass** die seitlichen Kompressionselemente derart geformt sind, dass sie unter den Bogenteilen des oberhalb folgenden Wirbels durchtreten können.

9. Stützanordnung (1) nach einem der Patentansprüche 1 bis 5, **dadurch gekennzeichnet, dass** die seitlichen Kompressiohselemente derart geformt sind, dass sie mit einer quer verlaufenden, interpedikulären Verbindungsstange verbunden werden können, die auf dem unterhalb folgenden Wirbel eingesetzt werden kann, insbesondere im Fall von Laminektomie.

10. Stützanordnung (1) nach einem der Patentansprüche 1 bis 5, **dadurch gekennzeichnet, dass** die seitlichen Kompressionselemente derart geformt sind, dass sie mit einer Verbindungsstange verbunden werden können, die mit einem Arthrodesesystem der beiden unterhalb folgenden Wirbel verbunden ist.

11. Stützanordnung (1) nach einem der Patentansprüche 1 bis 5, **dadurch gekennzeichnet, dass** die seitlichen Kompressionselemente (6) von den beiden seitlichen Teilen eines kreisförmigen Bandes (25) gebildet werden, das um die Dornfortsätze (9) der beiden behandelten Wirbel (2) gelegt ist.

12. Stützanordnung (1) nach einem der Patentansprüche 1 bis 11, **dadurch gekennzeichnet, dass** die seitlichen Übertragungsteile (7) aus Stegen (20) bestehen, die sich an den seitlichen Kompressionselementen (6) einerseits und am Block (5) andererseits abstützen.

13. Stützanordnung (1) nach einem der Patentansprüche 1 bis 11, **dadurch gekennzeichnet, dass** die seitlichen Übertragungsteile (7) aus Buckeln (30) bestehen, die entweder mit den seitlichen Kompressionselementen (6) oder mit dem Block (5) verbunden sind.
